Europäisches Patentamt

European Patent Office

Office européen des brevets

(11) Numéro de publication: **0 237 515**
**A2**

## (12) DEMANDE DE BREVET EUROPEEN

(21) Numéro de dépôt: **87870031.9**

(22) Date de dépôt: **05.03.87**

(51) Int. Cl.⁴: **C 07 K 3/18**

(30) Priorité: **07.03.86 US 836868**

(43) Date de publication de la demande:
**16.09.87 Bulletin 87/38**

(84) Etats contractants désignés:
**AT BE CH DE ES FR GB GR IT LI LU NL SE**

(71) Demandeur: **Smith Kline - RIT Société anonyme dite:**
**rue du Tilleul, 13**
**B-1320 Genval (Rixensart) (BE)**

(72) Inventeur: **Bollen, Alex Joseph**
**Gaasbeekstraat 68**
**B-1711 itterbeek (BE)**

**Chuchana, Paul**
**Les Cévennes Bât 1.2§949, avenue Louis Ravas**
**F-34100 Montpellier (FR)**

**Hoylaerts, Marc**
**Gemeentestraat 209**
**B-3200 Kessel-Lo (BE)**

(74) Mandataire: **Tasset, Gérard**
**SMITHKLINE - RIT rue de l'Institut, 89**
**B-1330 Rixensart (BE)**

(54) Purification de l'alpha-1-antiprotéase.

(57) Procédé de purification d'alpha-1-antiprotéase au départ d'une solution impure.

Le procédé comporte plusieurs stades au cours desquels la solution est passée sur une résine échangeuse d'ions et l'éluat est traité avec un adsorbant d'échange thiol-disulfure et ensuite avec de l'héparine avant d'être passé à nouveau sur un échangeur d'ions.

**Description**

Purification de l'alpha-1-antiprotéase

DOMAINE DE L'INVENTION

La présente invention concerne la purification de l'alpha-1-antiprotéase humaine, appelée ici alpha-1-antitrypsine.

Aperçu de l'arrière-plan technologique

L'alpha-1-antitrypsine (AAT) est une alpha-1-globuline présente dans le sérum et dans divers autres fluides de l'organisme. Telle qu'elle est synthétisée dans le foie, l'AAT mature est une glycoprotéine présentant un poids moléculaire d'environ 50.000 à 55.000 daltons. Parmi les enzymes protéolytiques inhibées par l'AAT, on peut citer la trypsine, la chymotrypsine, l'élastase pancréatique, la collagénase de la peau, la rénine et l'urokinase et des protéases de lymphocytes polynucléaires chez l'homme. Une déficience en AAT génétiquement acquise est associée à différents états pathologiques, notamment emphysème et maladie hépatique. A ce sujet, voir par exemple Morse, N. Eng. J. Med. 299 (19):1045-1048 (1978) et 299 (20):1099-1105 (1978); Tobin et al., Arch. Int. Med. 143 (7):1342-1348 (1982) et Carrell et al., Nature 298 (5872):329-334 (1982).

L'élastase est une protéinase qui détruit le tissu pulmonaire. Non détectée, son activité peut conduire à un emphysème. Par exemple, Gadek et al. Am. Rev. Respir. Dis. 127:S45 (1983) et Glaser et al. Am. Rev. Respir. Dis. 127:547-553 (1983) ont montré que l'AAT peut être thérapeutiquement utile dans le traitement de l'emphsème.

Etant donné l'activité thérapeutique de l'AAT et parce que certaines indications - comme la thérapie de remplacement chez les patients génétiquement déficients en AAT - en demandent des quantités relativement importantes, des chercheurs ont recherché des techniques de production d'AAT en grande quantité. Classiquement, de telles techniques impliquaient la purification d'AAT du plasma.

Des travaux récents se sont focalisés sur la production d'AAT de cellules ou microorganismes transformés, plus particulièrement E. coli et levures, étant donné les promesses de telles techniques pour la production de grandes quantités de produit au départ de gènes obtenus par ingéniérie. Plusieurs chercheurs ont fait état de succès dans de telles tentatives. Cependant, avant la présente invention, on n'avait pas connaissance d'un procédé pratique pour la purification d'une telle AAT recombinante utile en application pharmaceutique.

C'est un objet de la présente invention de fournir un procédé amélioré pour la purification d'AAT au départ de sérum ou de microorganismes recombinants, levures ou cellules, spécialement bactéries ou levures recombinantes, lequel procédé peut être appliqué à une grande échelle.

Laurell et al., J. Chromatog.278:53-61 (1983) décrivent l'usage de chromatographie d'échange à l'aide de thio-disulfure pour isoler l'AAT.

DESCRIPTION DETAILLEE DE L'INVENTION

Le procédé de l'invention comprend une suite d'étapes d'absorption dont chacune, individuellement, est effectuée suivant les techniques classiques de purification de protéines. Bien que les étapes individuelles soient, d'une manière générale, des techniques classiques de purification de protéines, ce sont des étapes particulières dans une séquence particulière d'étapes et de séquences pour obtenir un procédé de purification qui soit, à la fois, effectif et efficient.

L'AAT purifiée par le procédé de l'invention peut être identique à l'AAT du sérum. L'invention peut également être appliquée à différentes molécules d'AAT, c'est-à-dire des molécules d'AAT variant de l'AAT naturelle dans la structure secondaire ou tertiaire, comme cela peut être le cas pour certaines protéines AAT dérivées d'ADN recombinant. Des molécules d'AAT différentes dans la structure primaire peuvent également être utilisées pour autant que la capacité de l'AAT pour former des ponts di-sulfures au cours du stade d'échange thio-disulfure ne soit pas affectée contrairement et d'une manière significative. Comme molécules variantes d'AAT, on peut citer, par exemple, celles qui sont décrites par Courtney et al. dans EP-A-114777, par Courtney et al. dans Nature 313:149 (1985) et par Bollen et al. dans DNA 2:255 (1983).

Les stades du procédé de l'invention sont, de préférence, effectués sous la forme de stades de chromatographie, c'est-à-dire flux continu à travers un adsorbant, plutôt que par traitement en cuve. Les adsorbants utilisés dans le procédé de l'invention sont constitués par un support matriciel solide à travers lequel on fait passer différentes solutions d'AAT et on met ensuite en contact un ligand qui adsorbe soit l'AAT soit une ou plusieurs impuretés. Parmi les supports solides connus de ce genre, on peut citer le verre, la silice, l'alumine et la zirconite aussi bien que des supports organiques comme l'agarose, la cellulose, le dextranne, un polyamide, des copolymères polyacrylamide et vinyliques d'acrylates bifonctionnels avec divers monomères hydroxylés. Parmi les supports disponibles dans le commerce, on peut citer Affi-gel R, Sephadex R, résines HP telles que HP-20, XAD, Sepharose R et autres.

Le procédé s'applique à une solution impure d'AAT, c'est-à-dire une solution dans laquelle le niveau de pureté de l'AAT est inférieur à 25 % et typiquement inférieur à 10-15 %. Une telle solution peut être, par exemple, du sérum, un milieu de culture de bactérie productrice ou une autre culture cellulaire dans laquelle l'AAT est secrétée ou encore une levure hôte dans laquelle l'AAT est soluble. De préférence, un extrait brut de bactérie ou de levure est purifié partiellement par exemple par précipitation sélective au sulfate d'ammonium suivie de resolubilisation ou précipitation sélective des protéines contaminantes, par exemple par utilisation d'un polyalkylène glycol. Les techniques pour préparer du sérum contenant de l'AAT sont bien connues. Les techniques de préparation d'extraits bactériens ou d'autres cellules contenant la protéine soluble ou de solubilisation de la protéine dans de tels milieux sont également bien connues. Par exemple, des E. coli transformants sont recueillis par centrifugation et on provoque leur lyse par traitement à 0° C pendant 30 minutes dans un volume d'un dixième de tampon TES (50 mM Tris, pH 8, 5 mM EDTA et 25 % de saccharose) additionné de 0,5 mg/ml de lysozyme et 10 mg/ml d'ADNase. On ajoute alors du Triton X100 jusqu'à concentration finale de 0,1 %. La suspension est incubée à 0° C pendant 15 minutes et ensuite amenée à une concentration de 80 mM en Mg++ par addition de MgCl₂ molaire. Cette suspension est incubée pendant 5 minutes à 25° C. Après centrifugation à 15.000 tm pendant 10 minutes, on recueille le surnageant. On ajoute du sulfate d'ammonium au surnageant et on centrifuge la solution pendant 10 minutes à 10.000 tm. L'AAT est détectée dans la fraction à 50-75 % de sulfate d'ammonium. Ce précipité est redissous dans un tampon (50 mM Tris, pH 8, 25 mM NaCl) et dialysé contre le même tampon.

La première étape du procédé de l'invention est une étape d'échange ionique effectuée en présence d'un détergent doux pour éliminer les lipides et la plupart des acides nucléiques. Une colonne échangeuse d'ions, de préférence une colonne de type DEAE comme le DEAE-agarose, est équilibrée à un pH approximativement neutre, de préférence 6,5 avec un tampon phosphate contenant un peu de détergent. Après avoir fait passer la solution impure d'AAT à travers la colonne, la colonne est lavée avec le tampon d'équilibrage. L'AAT est alors éluée avec un sel, de préférence du NaCl environ 150-250 mM dans le tampon d'équilibrage. L'éluat salé est concentré par exemple par filtration ou par dialyse. On ajoute de l'éthylène diamine tétraacétate (EDTA) jusqu'à une concentration finale d'environ 20 mM et on amène le pH au dessus de la neutralité, par exemple au dessus de pH 7 jusqu'à environ pH 10, de préférence pH 8-9.

Dans la seconde étape, l'éluat concentré provenant de l'étape (1) est mis en contact avec un adsorbant d'échange thiol-disulfure. Un tel procédé utilisant des chaînes légères kappa (K) humaines immobilisées est décrit par Laurell et al., J. Chromatog. 278:53-61 (1983). L'AAT est élué des chaînes légères K fixées à un support solide à l'aide d'un agent réducteur, de préférence l'acide 4-nitrophényl-dithio-3,3'-dicarbonique et/ou le beta-mercaptoéthanol pour rompre les liaisons disulfure. L'éluat est concentré, désalé et ajusté à pH 7-8.

L'éluat concentré de l'étape (2) est alors mis en contact avec de l'héparine fixée à un support solide, par exemple une colonne héparine-agarose, à une force ionique permettant d'adsorber la plupart des lipoprotéines résiduelles. Ce qui est passé à travers la colonne, c'est-à-dire l'effluent de cet adsorbant, contient l'AAT.

Dans la quatrième étape, l'effluent du stade héparine est mis en contact avec des ions Zn++ immobilisés, par exemple, une colonne de chelate de zinc, de préférence une colonne d'agarose, afin d'adsorber sélectivement l'AAT. L'AAT est éluée en abaissant le pH en dessous de 6, par exemple 5,5 ou par élutions successives avec de l'histidine. Après élution acide, le pH est rapidement réajusté à peu près à neutralité, c'est-à-dire à pH 6,5-7,5. L'éluat est alors dialysé pour éliminer les agents d'élution.

Dans la cinquième étape, la solution d'AAT est mise en contact avec un second adsorbant échangeur d'ions, de préférence une colonne d'aminohexyl agarose. L'AAT est éluée sélectivement en utilisant un gradient de sel (150-250 mM NaCl). L'AAT pur éluée est désalée par exemple par dialyse et concentrée, de préférence par lyophilisation.

Si on le désire, l'AAT résultant de ce procédé peut être soumise à des stades ultérieurs de purification pour éliminer des traces de contaminants, par exemple par chromatographie d'affinité en employant, par exemple, de l'anhydrochymotrypsine immobilisée ou des anticorps anti-AAT, ou anti-protéines contaminantes immobilisées.

EXEMPLES

Les exemples qui suivent sont purement illustratifs et ne sont pas limitatifs à un procédé de purification préféré de l'invention.

Exemple 1

Purification d'AAT mature d'origine levure

Des plasmides recombinants exprimant l'AAT humaine mature sous le contrôle du promoteur Arg3 de levure, tel que décrits précédemment par Cabezon et al., Proc. Natl. Acad. Sci. USA 81:6594-6598 (1984) ont été utilisés pour exprimer l'AAT dans les souches S. cerevisiae 10S442 déficientes en peptidase (leu 2-3, leu 2-112, pep 4-3) et TCY1 (ura3, leu2 défectives).

Précipitation préliminaire : Après destruction mécanique des membranes cellulaires d'environ 1,5 à 2 kg de levure dans un appareil Dyno-Mill refroidi à l'éthanol-carboglace à 3000 tm (vitesse de pompage : environ 5 l/h), en présence de 20 mM de n-mercaptoéthanol, 5 mM EDTA, l mM PMSF et l mM benzamidine (volume total compris entre 2,0 et 2,5 l) dans du tampon Tris-HCl 50 mM à pH 8,0, le pH de l'extrait brut a été ajusté à 6,5 avec HCl 1N, sous agitation. On y a ajouté du polyéthylène glycol 1000 jusqu'à une concentration de 7 %. Après deux heures d'incubation, l'extrait brut a été centrifugé à 16.000 g pendant 5 heures. Les membranes de levure précipitées (environ 500 ml) ont été mises en suspension par sonication dans un litre de tampon phosphate 20 mM pH 6,5 contenant 7 % de polyéthylène glycol (PEG) 1000 et recentrifugées pendant 5 heures au moins. Les deux surnageants ont été rassemblés (environ 3 litres), on y a ajouté du Triton WR1339 jusqu'à concentration de 0,1 % et le pH a été ajusté à 6,5. La force ionique totale a été mesurée et ajustée à 70 mM en unités d'équivalents NaCl, par dilution à l'aide d'eau distillée.

Chromatographie DEAE

L'extrait contenant l'AAT (ici $\alpha_1$-PI) partiellement épuisé en organelles cellulaires et en lipoprotéines a alors été mis sur une colonne préparative de Sepharose-DEAE à flux rapide (5 x 26 cm) équilibrée dans un tampon phosphate (20 mM) à pH 6,5 contenant 0,1 % de Triton et 0,01 % de NaN$_3$, à une vitesse de passage de 600 ml/h. La colonne a été lavée avec le tampon de départ jusqu'à ce que l'absorbance (280 nm) de l'effluent tombe en dessous de 2,0 (approximativement 10 l), après quoi une fraction protéinique contenant l'$\alpha_1$-PI a été éluée par fractions avec du NaCl 150 mM ajouté au tampon de départ. Les composés résiduels fixés ont été élués par fractions respectivement avec NaCl lM et NaOH 0,5M, après quoi la colonne a été rééquilibrée avec le tampon de départ. Après concentration de la fraction $\alpha_1$-PI jusqu'à 400 ml, on a ajouté EDTA jusqu'à concentration 20 mM et le pH a été amené à 8,5 avec du Tris en poudre.

Chromatographie échange thiol

Le matériau légèrement enrichi obtenu à la fin de l'étape précédente a été mis sur une résine constituée de chaînes légères d'immunoglobuline humaine, idiotype K, insolubilisées sur Sepharose 4B, suivi d'un couplage avec du bromure de cyanogène.

Comme indiqué plus haut, des chaînes légères K humaines peuvent être isolées de l'urine de patients souffrant de myélome sujets au syndrome de Bence-Jones. Après chargement de lots d'urine concentrée sur colonnes DEAE-Sepharose, les chaînes K ont été incubées avec des concentrations saturantes d'acide bis-(4-nitrophényl)dithio-3,3'-dicarbonique (DTNB) dans un tampon Tris-HCl 1M à pH 8,5, contenant EDTA 100 mM, pendant 4 jours à température ordinaire. Ce procédé dissocie effectivement les dimères qui apparaissent lors de formation d'un pont disulfure entre les cystéines C-terminales présentes dans les chaînes K et introduit un groupe protecteur provenant des fonctions thiol. Après séparation des DTNB et TNB en excès par chromatographie sur Sephadex G-25, les chaînes K protégées ont été fixées à 500 ml de Sepharose 4B et activées avec du CNBr immédiatement avant d'être mises en oeuvre. De cette manière, 0,4 mole de thiol ont été introduits par ml de gel, comme calculé au départ de l'absorbance (412 nm) du TNB séparé de la résine après réduction complète des ponts disulfure à l'aide de $\beta$-mercapto éthanol ($\beta$-SH).

La solution d'$\alpha_1$-PI a été passée sur une colonne de chaînes K insolubilisées (2,6 x 94 cm) équilibrée dans un tampon Tris/HCl (50 mM) contenant NaCl (20 mM), EDTA (20 mM), Triton (0,1 %) et NaN$_3$ (0,01 %) à une vitesse de passage de 100 ml/h. La fraction qui est passée à travers la colonne a été traitée par $\beta$-SH (20 mM) pour réduire les ponts disulfures impliquant TNB et les protéines solubles et a été dialysée contre le tampon de chromatographie auquel on a ajouté du charbon actif pour accélérer l'élimination de TNB. Cette fraction qui est passée à travers la colonne (non entièrement épuisée en $\alpha_1$-PI) a ensuite été repassée sur la colonne, comme décrit pour le premier passage.

Après chaque passage, la colonne a été lavée avec le tampon de départ et la fraction protéinique contenant $\alpha_1$-PI a été éluée spécifiquement avec un excès de TNB (1 mg/ml de DTNB + 0,25 mg/ml de dithiothréitol dans le tampon de départ), ce qui inverse l'équilibre -S-S-TNB + Protéine SH $\leftrightarrows$ -S-S-P + TNB-SH, et par là détache certaines protéines, parmi lesquelles $\alpha_1$-PI.

Une élution complète a ensuite été réalisée par réduction complète des ponts disulfures à l'aide de $\beta$-SH 20 mM ajouté au tampon de départ. Avant usage, la colonne a été réactivée par passage de DTNB dans le tampon de départ (1 mg/ml).

La fraction contenant $\alpha_1$-PI spécifiquement éluée a été réduite par $\beta$-SH 20 mM et dialysée contre du tampon phosphate 20 mM à pH 7,5 auquel on a ajouté du charbon actif.

Alternativement après réduction, l'éluat a été concentré jusqu'à 150 ml dans un appareil à concentrer du type Amicon DC2 et désalé par chromatographie sur Sephadex G-25 dans une colonne (5 x 50 cm) équilibrée avec du tampon phosphate 20 mM à pH 7,5.

Chromatographie héparine-agarose

Le dialysat de la solution d'$\alpha_1$-PI désalée par chromatographie a été mis sur une colonne héparine-agarose (2,6 x 36 CM) équilibrée dans du tampon phosphate 20 mM à pH 7,5 et lavée avec du tampon de départ à une vitesse d'écoulement de 70 ml/h. Les fractions qui, à ce stade, sont passées à travers la colonne sont rassemblées et ensuite mises en oeuvre. Les protéines fixées sur la colonne ont été éluées avec du NaCl 1M et écartées. A chaque cinquième cycle, une solution de NaSCN (2M) a été passée pour détacher les molécules adhérant à l'agarose par interactions non spécifiques.

Chromatographie au chelate de zinc

La fraction qui est passée à travers la colonne à l'étape précédente est mise en dépôt soit comme pool de fractions recueillies soit directement en connectant la sortie de la colonne héparine-agarose à l'entrée d'une colonne de Sepharose de chelation (2,6 x 19 cm) chargées d'ions Zn++ et équilibrée avec du tampon phosphate 20 mM, pH 7,5. Après avoir chargé complètement la colonne, celle-ci a été lavée avec du tampon phosphate 25 mM, pH 6,5 contenant 0,5M NaCl. On a obtenu l'élution spécifique d'$\alpha_1$-PI en ordre principal en abaissant le pH à 5,5 ou par élution par gradient avec de l'histidine (0 - 25 mM) dans le tampon de lavage. Cette opération a été suivie d'une élution non-sélective à l'aide d'EDTA 50 mM dans un tampon Tris-HCl 0,2M à pH 8,0, après quoi la colonne a été ré-équilibrée avec ZnCl$_2$. Dans le cas d'élution acide, le pH a été immédiatement amené à environ 7 avec 1 ml de tampon Tris-HCl 1M à pH 8,0, présent dans les tubes pendant la collecte de l'éluat. Les éluats rassemblés ont ensuite été dialysés contre un tampon phosphate 25 mM à pH 6,5, 25 mM en NaCl.

## Chromatographie AH-Sepharose

La solution d'$\alpha_1$-PI encore légèrement contaminée a été chargée sur une colonne d'amino-hexyl agarose (2,6 x 18 cm) (AH-Sepharose, Pharmacia, Stockholm, Suède) utilisée comme échangeuse d'ions et équilibrée dans du tampon phosphate 25 mM à pH 6,5, 25 mM en NaCl. Les protéines adsorbées ont été éluées par un volume total de 600 ml d'un gradient linéaire de NaCl (25 à 300 mM) dans le tampon de départ, à une vitesse d'écoulement de 50 ml/h. Les fractions contenant $\alpha_1$-PI à l'état pur ont été raseemblées et dialysées contre un tampon phosphate 5 mM à pH 7, 15 mM en NaCl et lyophilisées. Après dissolution de l'$\alpha_1$-PI purifiée dans de l'eau distillée dans un dixième du volume initial avant lyophilisation, des échantillons dix fois concentrés ont été obtenus; ils présentaient une force ionique adéquate pour le maintien de l'activité biologique.

## Exemple 2

### Purification et activité de différentes molécules d'AAT

Les molécules d'AAT suivantes ont été purifiées en substance par le procédé décrit dans l'Exemple 1 :
1. AAT dépourvue d'acides aminés en position 5N terminale et exprimée dans E. coli;
2. AAT mature (ayant N-met à la place de N-glu et sans ala en position 8) exprimée dans E. coli;
3. AAT mature (ayant N-met à la place de N-glu) exprimée dans une levure;
4. AAT de plasma humain.

Des E. coli transformants ont été soumis à une lyse pour préparer des extraits cellulaires desquels l'AAT a été précipitée avec du sulfate d'ammonium, en substance comme décrit plus haut dans la description.

L'AAT a été obtenue du plasma par des techniques classiques.

L'activité des quatre protéines purifiées par la méthode de l'invention a été comparée à celle d'une préparation d'AAT classique obtenue de sérum, dans un test classique d'inhibition de la trypsine. Le test mesure la capacité d'inhibition de l'$\alpha_1$-antitrypsine vis-à-vis de la trypsine. Il consiste en un microtest qui utilise le substrat chromogène S2444 (L-pyroglutamylglycyl-L-arginine, chlorhydrate de p-nitroanilide, Kabi Diagnostica, Stockholm, Suède). Les plaques microtest en polystyrène ont été incubées pendant 1 heure avec 1 % d'albumine sérique bovine et lavées abondamment à l'eau distillée. L'$\alpha_1$-antitrypsine à différentes concentrations a été incubée avec une quantité fixe de trypsine pendant 20 minutes à 37° C dans un volume de réaction final de 100 µl de mélange de Tris HCl 0,1 M pH 8,2 / NaCl 0,15M / EDTA 0,01 M / polyéthylène glycol PM 6000, 0,5 %. Des échantillons ont été lentement refroidis jusqu'à température ordinaire et exposés ensuite au substrat chromogène (20 µl d'une solution aqueuse de S2444 0,003M). Après 5 minutes à température ordinaire, la réaction a été stoppée avec 50 µl d'acide acétique à 50 %.

L'adsorbance a été lue à 405 nm dans un lecteur automatique de micro-ELISA (DYNATECH AM120). Des courbes d'étalonnage ont été dressées à la fois pour la trypsine et pour l'$\alpha_1$-antitrypsine. Les échantillons à tester ont été dilués en série dans le tampon de réaction.

Les résultats qui sont rapportés dans le Tableau suivant démontrent que le procédé de l'invention peut être utilisé pour purifier l'AAT de différentes sources, entre autres diverses molécules d'AAT, sans affecter contrairement son activité d'inhibition de protéase. Les échantillons 1, 2, 3 et 4 sont ceux qui sont définis plus haut.

## Test d'activité d'AAT purifiée produite dans des microorganismes ou au départ de collecte de plasma humain

| Concentration en AAT (µg/ml) | Densité optique ($A_{410}$) (inhibition de l'activité trypsine) | | | | |
|---|---|---|---|---|---|
| | standard | 1 | 2 | 3 | 4 |
| 0 | .95(0) | 1.00(0) | 1.02(0) | 1.02(0) | 0.90(0) |
| 0.25 | – – | 0.56(44) | – – | 0.95(7) | 0.74(17) |
| 0.50 | .78(11) | 0.16(84) | 0.9(10) | 0.84(17) | – – |
| 1 | .63(33) | 0.05(95) | – – | – – | 0.48(46) |
| 1.50 | – – | – – | 0.47(54) | 0.54(47) | – – |
| 2 | .32(66) | – – | – – | – – | 0.06(93) |
| 2.50 | .24(74) | | | | |
| 3.50 | .17(82 | | | | |
| 4.50 | .1 (90) | | | | |

N.B. Le pourcentage de l'inhibition de l'activité trypsine est indiqué entre parenthèses.

La description et les exemples ci-dessus sont une description complète de l'invention impliquant ses formes préférentielles. L'invention n'est cependant pas limitée à la construction qui y est précisément décrite, mais comme plutôt toutes ses modifications et variantes qui sont comprises dans la portée des revendications qui suivent.

**Revendications**

1. Procédé de purification d'AAT d'une solution impure la contenant caractérisé en ce que
(1) la solution est mise en contact avec un adsorbant échangeur d'ions et l'AAT en est éluée;
(2) l'éluat de l'étape (1) est mis en contact avec un adsorbant d'échange thiol-disulfure et l'AAT en est éluée;
(3) l'éluat de l'étape (2) est mis en contact avec de l'héparine fixée sur un support solide;
(4) l'effluent de l'étape (3) est mis en contact avec un adsorbant du type chelate de zinc et on en élue l'AAT;
(5) l'éluat de l'étape (4) est mis en contact avec un adsorbant échangeur d'ions et on en élue l'AAT.

2. Procédé suivant la revendication 1 caractérisé en ce que chaque étape est effectuée suivant le mode chromatographique et dans lequel.
à l'étape (1), la solution est mise en contact avec une colonne échangeuse d'ions du type DEAE en présence d'un détergent et on en élue l'AAT avec du NaCl 150-250 mM
à l'étape (2), l'éluat du stade (1) est mis en contact avec une colonne de chaîne légère K humaine immobilisée et on en élue l'AAT à l'aide d'un agent réducteur
à l'étape (4), l'effluent de l'étape (3) est mis en contact avec une colonne de chélate de zinc et on en élue l'AAT soit par abaissement du pH en dessous de 6 soit en mettant l'AAT liée en contact avec de l'histidine et en ajustant immédiatement le pH à 6,5-7,5 et

à l'étape (5), l'éluat de l'étape (3) est mis en contact avec une colonne échangeuse d'ions du type aminohexyle et on en élue l'AAT à l'aide de NaCl (150 mM } 250 mM).